# EUROPEAN PATENT APPLICATION

(11) **EP 4 230 743 A1**
(43) Date of publication of application: **23.08.2023**
(21) Application number: 21895150.7
(22) Date of filing: 19.11.2021
(51) Int. Cl.: C12N 15/77, C12N 9/88, C12P 13/14

(54) **MICROORGANISM HAVING ENHANCED L-GLUTAMINE PRODUCING ABILITY, AND L-GLUTAMINE PRODUCING METHOD USING SAME**

(30) Priority: 20.11.2020 KR 20200156903
(71) Applicant: CJ Cheiljedang Corporation, Seoul 04560 (KR)
(72) Inventor: CHOI, Su Jin, Seoul 04560 (KR); YANG, Sunyoung, Seoul 04560 (KR); LEE, Kwang Woo, Seoul 04560 (KR)
(74) Representative: Heubeck, Christian
(86) International application number: PCT/KR2021/017074
(87) International publication number: WO 2022/108383

(57) **Abstract**

The present disclosure relates to a microorganism having enhanced L-glutamine production ability and an L-glutamine production method using the same.

## Description

### [Technical Field]

The present disclosure relates to a microorganism having enhanced L-glutamine production ability and an L-glutamine production method using the same.

### [Background Art]

L-glutamine is a widely used amino acid in various industries, including pharmaceuticals, cosmetics, and health foods. It is commonly utilized as a therapeutic agent for digestive disorders, a liver function enhancer, a brain function enhancer, an immune enhancer, a therapeutic agent for gastric ulcer, a therapeutic agent for alcoholism, a moisturizer in cosmetics, an exercise nutritional supplement, and a patient nutritional supplement.

Representatively, *Corynebacterium glutamicum* and *Escherichia coli* are used for L-glutamine production through microorganisms. As the L-glutamine biosynthesis pathway, α-keto glutaric acid produced through glycolysis and TCA cycle (tricarboxylic acid cycle) is used as a precursor to produce L-glutamate by glutamate dehydrogenase, and L-glutamine is finally produced through the reaction of glutamine synthetase (Production of glutamate and glutamate-related amino acids: Molecular Mechanism Analysis and Metabolic Engineering, Amino acid Biosynthesis-pathways, regulation and metabolic engineering pp1-38).

However, in accordance with the increase in demand for L-glutamine, studies to effectively increase the L-glutamine production ability are still required.

### [Disclosure]

### [Technical Problem]

As a result of efforts to increase L-glutamine production ability, the present inventors have discovered that L-glutamine is highly efficiently produced in microorganisms containing phosphoenolpyruvate carboxykinase, and thus completed the present disclosure.

### [Technical Solution]

An object of the present disclosure is to provide a microorganism of the genus *Corynebacterium* having phosphoenolpyruvate carboxykinase activity weakened and L-glutamine production ability.

Another object of the present disclosure is to provide an L-glutamine production method including culturing a microorganism of the genus *Corynebacterium* having phosphoenolpyruvate carboxykinase activity weakened and L-glutamine production ability in a medium.

Still another object of the present disclosure is to provide a method for producing a microorganism for L-glutamine production, including weakening phosphoenolpyruvate carboxykinase.

Still another object of the present disclosure is to provide a composition for L-glutamine production containing a microorganism in which phosphoenolpyruvate carboxykinase is weakened; a medium in which the microorganism has been cultured; or a combination thereof.

Still another object of the present disclosure is to provide use of a microorganism of the genus *Corynebacterium* for L-glutamine production, in which phosphoenolpyruvate carboxykinase is weakened.

### [Advantageous Effects]

The microorganism according to the present disclosure, which contains weakened phosphoenolpyruvate carboxykinase, can produce L-glutamine highly efficiently. The prepared L-glutamine can be utilized in various products, including animal feed or animal feed additives as well as human food or food additives, and pharmaceuticals.

### [Detailed Description of the Invention]

Each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description below.

Further, those skilled in the art will recognize, or be able to ascertain using no more than routine experimentation, many equivalents to the specific embodiments of the present disclosure described herein. Further, these equivalents should be interpreted to fall within the scope of the present invention. In addition, a number of papers and patent documents are referenced throughout the present specification and their citations are indicated. The contents of the cited papers and patent documents are incorporated herein by reference in their entirety to more clearly describe the level of the technical field to which the present disclosure belongs and the contents of the present disclosure.

An aspect of the present disclosure for achieving the objects provides a microorganism of the genus *Corynebacterium* having phosphoenolpyruvate carboxykinase activity weakened and L-glutamine production ability.

As used herein, the term "phosphoenolpyruvate carboxykinase (PEPCK; EC 4.1.1.32)" refers to an enzyme that is involved in the gluconeogenesis process and converts oxaloacetate into phosphoenolpyruvate and carbon dioxide in the presence of GTP. Phosphoenolpyruvate carboxykinase of the present disclosure may be used interchangeably with PEPCK.

The amino acid sequence of PEPCK may be acquired from a known database such as NCBI's Genebank.

For example, PEPCK of the present disclosure may be a protein derived from a microorganism of the genus *Corynebacterium.* More specifically, PEPCK of the present disclosure may be derived from *Corynebacterium glutamicum, Corynebacterium deserti, Corynebacterium crudilactis, Corynebacterium efficiens, Corynebacterium callunae* and the like, but is not limited thereto.

PEPCK of the present disclosure may be an endogenous protein of the microorganism of the genus *Corynebacterium* of the present disclosure.

In the present disclosure, PEPCK of the present disclosure may have, contain, or consist of the amino acid sequence set forth in SEQ ID NO: 1; or may essentially consist of the amino acid sequence.

In the present disclosure, the amino acid sequence of SEQ ID NO: 1 may include an amino acid sequence having at least 70%, 75%, 80%, 85%, 90%, 95%, 96%, 97%, 98%, 99%, 99.5%, 99.7% or 99.9% or more homology or identity thereto. In addition, it is obvious that a protein having an amino acid sequence in which some sequences are deleted, modified, substituted, conservatively substituted, or added is also included within the scope of the present disclosure, as long as it is an amino acid sequence that has such homology or identity and exhibits an efficacy corresponding to that of the protein including the amino acid sequence of SEQ ID NO: 1.

Examples thereof include a case of having sequence additions or deletions, naturally occurring mutations, silent mutations, or conservative substitutions that do not alter the function of the protein of the present disclosure at the N-terminus, C-terminus of and/or inside the amino acid sequence.

The "conservative substitution" refers to the substitution of one amino acid with another amino acid having similar structural and/or chemical properties. Such amino acid substitution may generally occur based on similarities in polarity, charge, solubility, hydrophobicity, hydrophilicity and/or amphipathic nature of the residues. Typically, conservative substitutions may have little or no effect on the activity of the protein or polypeptide.

As used herein, the term "homology" or "identity" refers to the degree of similarity between two given amino acid sequences or nucleotide sequences and may be expressed as a percentage. The terms homology and identity are often used interchangeably.

Sequence homology or identity of conserved polynucleotides or polypeptides is determined by standard alignment algorithms, and a default gap penalty established by the program being used may be utilized together. Substantially homologous or identical sequences are generally capable of hybridizing with all or part of the sequence under moderate or stringent conditions. It is obvious that hybridization also includes hybridization with polynucleotides containing common codons or codons in consideration of codon degeneracy in polynucleotides.

Whether any two polynucleotide or polypeptide sequences have homology, similarity or identity may be determined using known computer algorithms such as the "FASTA" program using default parameters as in, for example, Pearson et al., (1988) [Proc. Natl. Acad. Sci. USA 85]: 2444. Alternatively, homology, similarity or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information Database, or ClustalW may be used to determine homology, similarity or identity.

Homology, similarity or identity of polynucleotides or polypeptides may be determined by comparing sequence information using, for example, a GAP computer program such as Needleman et al. (1970), J Mol Biol. 48: 443, for example, as known in Smith and Waterman, Adv. Appl. Math (1981) 2: 482. In summary, the GAP program may define homology, similarity or identity by the value acquired by dividing the total number of symbols in the shorter of two sequences by the number of similarly arranged symbols (i.e., nucleotides or amino acids). Default parameters for the GAP program may include (1) a binary comparison matrix (containing values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or gap opening penalty of 10, gap extension penalty of 0.5); and (3) no penalty for end gaps.

PEPCK of the present disclosure may be encoded by the pck gene. The pck gene of the present disclosure may include all genes known to encode proteins having PEPCK activity.

Specifically, the pck gene of the present disclosure may be pck derived from a microorganism of the genus *Corynebacterium.* More specifically, the pck gene of the present disclosure may be pck derived from a *Corynebacterium glutamicum* strain. For example, the pck gene of the present disclosure may be a polynucleotide encoding WP_011015446.1 derived from *Corynebacterium glutamicum* ATCC13032.

As used herein, the term "polynucleotide" is a DNA or RNA strand having a certain length or more as a polymer of nucleotides in which nucleotide monomers are covalently linked in a long chain shape, and more specifically means a polynucleotide fragment encoding the protein.

The polynucleotide encoding PEPCK of the present disclosure may include a nucleotide sequence encoding the amino acid sequence set forth in SEQ ID NO: 1. As an example of the present disclosure, the polynucleotide of the present disclosure may have or include the nucleotide sequence of SEQ ID NO: 2. In addition, the polynucleotide of the present disclosure may consist of or essentially consist of the sequence of SEQ ID NO: 2. Specifically, the PEPCK may be encoded by a polynucleotide set forth in the nucleotide sequence of SEQ ID NO: 2.

In the polynucleotide of the present disclosure, various modifications may be made to the coding region within the range of not changing the amino acid sequence of PEPCK in consideration of codon degeneracy or preferred codons in organisms intended to express PEPCK of the present disclosure. Specifically, the polynucleotide of the present disclosure has or includes a nucleotide sequence having 70% or more, 75% or more, 80% or more, 85% or more, 90% or more, 95% or more, 96% or more, 97% or more or 98% or more homology or identity to the sequence of SEQ ID NO: 2, or may consist of or essentially consist of a nucleotide sequence having 70% or more, 75% or more, 80% or more, 85% or more, 90% or more, 95% or more, 96% or more, 97% or more or 98% or more homology or identity to the sequence of SEQ ID NO: 2, but is not limited thereto.

In addition, the polynucleotide of the present disclosure may include any probe that may be prepared from a known gene sequence, for example, any sequence that may hybridize with a sequence complementary to all or part of the polynucleotide sequence of the present disclosure under stringent conditions without limitation. The "stringent condition" refers to conditions that allow specific hybridization between polynucleotides. These conditions are specifically described in literatures (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having high homology or identity hybridize, for example, polynucleotides having 70% or more, 75% or more, 76% or more, 85% or more, 90% or more, 95% or more, 96% or more, 97% or more, 98% or more or 99% or more homology or identity hybridize, and polynucleotides having lower homology or identity than this do not hybridize, or a condition in which washing is performed one time, specifically two to three times, at a salt concentration and temperature corresponding to 60°C, 1XSSC, 0.1% SDS, specifically 60°C, 0.1XSSC, 0.1% SDS, more specifically 68°C, 0.1XSSC, and 0.1% SDS, which is the washing condition of conventional Southern hybridization.

Hybridization requires that two nucleic acids have complementary sequences, although mismatches between bases are possible depending on the stringency of hybridization. The term "complementary" is used to describe the relation between nucleotide bases that are capable of hybridizing to each other. For example, with regard to DNA, adenine is complementary to thymine and cytosine is complementary to guanine. Hence, the polynucleotide of the present disclosure may also include substantially similar nucleic acid sequences as well as isolated nucleic acid fragments complementary to the entire sequence.

Specifically, a polynucleotide having homology or identity to the polynucleotide of the present disclosure may be detected using hybridization conditions including a hybridization step at a Tm value of 55°C and using the above-described conditions. In addition, the Tm value may be 60°C, 63°C or 65°C, but is not limited thereto and may be appropriately adjusted by those skilled in the art according to the purpose.

Appropriate stringency for hybridizing the polynucleotides depends on the length of the polynucleotides and the degree of complementarity, and the parameters are well known in the art (e.g., J. Sambrook et al., supra).

In the present disclosure, the term "microorganism (or strain)" includes both wild-type microorganisms and naturally or artificially genetically modified microorganisms, is a microorganism whose specific mechanism is weakened or enhanced by causes such as insertion of an external gene or enhancement or weakening of the activity of an endogenous gene, and may be a microorganism that has undergone genetic modification to produce a desired polypeptide, protein or product.

In the present disclosure, the term "weakening" of the activity of a polypeptide (including, for example, proteins specified by the name of each enzyme) is a concept that includes both decreased activity compared to intrinsic activity or no activity. The weakening may be used interchangeably with terms such as inactivation, deficiency, down-regulation, decrease, reduction, and attenuation.

The weakening may also include a case where the activity of the polypeptide itself is decreased or eliminated compared to the activity of the polypeptide originally possessed by the microorganism by mutation of the polynucleotide encoding the polypeptide, and the like, a case where the overall activity degree and/or concentration (expression level) of polypeptide in the cell is lower than that of the native strain due to inhibition of expression of the gene of the polynucleotide encoding the polypeptide or inhibition of translation into a polypeptide, a case where the expression of the polynucleotide is not achieved at all, and/or a case where the activity of the polypeptide is not exhibited even though the polynucleotide is expressed.

Such weakening of the activity of a polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al., Molecular Cloning 2012, and the like).

Specifically, weakening of the activity of the polypeptide of the present disclosure may be
1) deletion of all or part of the gene encoding the polypeptide;
2) modification of the expression control region (or expression control sequence) to decrease the expression of the gene encoding the polypeptide;
3) modification of the amino acid sequence constituting the polypeptide (e.g., deletion/substitution/addition of one or more amino acids on the amino acid sequence) so that the activity of the polypeptide is eliminated or weakened;
4) modification of the nucleotide sequence encoding the polypeptide so that the activity of the polypeptide is eliminated or weakened (e.g., deletion/substitution/addition of one or more nucleotides on the nucleotide sequence of the gene encoding the polypeptide to encode a modified polypeptide so that the activity of the polypeptide is eliminated or weakened);
5) modification of the nucleotide sequence encoding the initiation codon or 5'-UTR region of a gene transcript encoding the polypeptide;
6) introduction of an antisense oligonucleotide (e.g., antisense RNA) that binds complementarily to the transcript of the gene encoding the polypeptide;
7) addition of a sequence complementary to the Shine-Dalgarno sequence at the front end of the Shine-Dalgarno sequence of the gene encoding the polypeptide to form a secondary structure to which ribosomes cannot be attached;
8) addition of a promoter transcribed in the opposite direction to the 3' end of the open reading frame (ORF) of the nucleotide sequence encoding the polypeptide (reverse transcription engineering, RTE); or
9) a combination of these (e.g., a combination of two or more selected from 1) to 8)), but is not particularly limited thereto.

For example,
1) The deletion of all or part of the gene encoding the polypeptide may be removal of the entire polynucleotide encoding the endogenous target polypeptide in the chromosome, replacement with a polynucleotide in which some nucleotides are deleted, or replacement with a marker gene.
2) The modification of the expression control region (or expression control sequence) may be occurrence of mutation in the expression control region (or expression control sequence) by deletion, insertion, non-conservative or conservative substitution, or a combination thereof, or replacement with a sequence having weaker activity. The expression control region includes, but is not limited to, a promoter, an operator sequence, a sequence encoding a ribosome binding site, and a sequence regulating termination of transcription and translation.
3) and 4) The modification of the amino acid sequence or nucleotide sequence may be occurrence of sequence mutation in the amino acid sequence of a polypeptide or a polynucleotide sequence encoding the polypeptide by deletion, insertion, non-conservative or conservative substitution, or a combination thereof so that the activity of the polypeptide is weakened, or replacement with an amino acid sequence or polynucleotide sequence improved to have weaker activity or an amino acid sequence or polynucleotide sequence improved not to exhibit activity, but is not limited thereto. For example, expression of a gene may be inhibited or weakened by introducing a mutation into a polynucleotide sequence to form a termination codon, but is not limited thereto.
5) The modification of the nucleotide sequence encoding the initiation codon or 5'-UTR region of a gene transcript encoding the polypeptide may be, for example, substitution with a nucleotide sequence encoding another initiation codon having a lower polypeptide expression rate than the endogenous initiation codon, but is not limited thereto.
6) The introduction of an antisense oligonucleotide (e.g., antisense RNA) that binds complementarily to the transcript of the gene encoding the polypeptide may be referred to, for example, [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence at the front end of the Shine-Dalgarno sequence of the gene encoding the polypeptide to form a secondary structure to which ribosomes cannot be attached may be to disable or slow down mRNA translation.

In addition, 8) the addition of a promoter transcribed in the opposite direction to the 3' end of ORF of the nucleotide sequence encoding the polypeptide may be to weaken the activity by making an antisense nucleotide complementary to the transcript of the gene encoding the polypeptide.

The modification of part or all of the polynucleotides in the microorganism of the present disclosure may be induced by (a) homologous recombination using a vector for chromosomal insertion into a microorganism or genome editing using engineered nucleases (e.g., CRISPR-Cas9) and/or (b) treatment with light such as ultraviolet light and radiation and/or chemicals, but is not limited thereto. The method for modifying part or all of the gene may include a method by DNA recombination technology. For example, deletion of part or all of a gene may be achieved by injecting a nucleotide sequence or vector containing a nucleotide sequence homologous to the target gene into the microorganism to cause homologous recombination. The injected nucleotide sequence or vector may include a dominant selection marker, but is not limited thereto.

The vector of the present disclosure may include a DNA product containing the nucleotide sequence of a polynucleotide encoding the target polypeptide operably linked to a suitable expression control region (or expression control sequence) so that the target polypeptide is expressed in a suitable host. The expression control region may include a promoter capable of initiating transcription, an arbitrary operator sequence for regulating such transcription, a sequence encoding a suitable mRNA ribosome binding site, and a sequence regulating termination of transcription and translation. After transformation into a suitable host cell, the vector may replicate or function independently of the host genome and may integrate into the genome itself.

Vectors used in the present disclosure are not particularly limited, and any vectors known in the art may be used. Examples of commonly used vectors include natural or recombinant plasmids, cosmids, viruses and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescript II-based, pGEM-based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, pCC1BAC vectors and the like may be used.

For example, a polynucleotide encoding the target polypeptide may be inserted into a chromosome through a vector for chromosomal insertion into a cell. Insertion of the polynucleotide into the chromosome may be achieved by any method known in the art, for example, homologous recombination, but is not limited thereto. A selection marker for determining whether the chromosome is inserted may be additionally included. The selection marker is used to select cells transformed with a vector, that is, to determine whether the target nucleic acid molecule has been inserted, and markers conferring a selectable phenotype such as drug resistance, auxotrophy, resistance to cytotoxic agents or expression of surface polypeptides may be used. In an environment treated with a selective agent, only cells expressing the selection marker survive or exhibit other expression traits, and thus transformed cells may be selected.

As used herein, the term "transformation" means introducing a vector containing a polynucleotide encoding the target polypeptide into a host cell or microorganism so that the polypeptide encoded by the polynucleotide can be expressed in the host cell. As long as the transformed polynucleotide can be expressed in the host cell, it may be inserted into and located in the chromosome of the host cell or located outside the chromosome. In addition, the polynucleotide includes DNA and/or RNA encoding the target polypeptide. The polynucleotide may be introduced in any form as long as it can be introduced into and expressed in a host cell. For example, the polynucleotide may be introduced into a host cell in the form of an expression cassette, which is a genetic construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replication. In addition, the polynucleotide may be introduced into a host cell in its own form and operably linked to a sequence required for expression in the host cell, but is not limited thereto.

In addition, the term "operably linked" means that the polynucleotide sequence is functionally linked to the promoter sequence that initiates and mediates transcription of the polynucleotide encoding the target polypeptide of the present disclosure.

The microorganism of the present disclosure may be a microorganism having increased L-glutamine production ability compared to that of a parent strain or a wild-type strain of the genus *Corynebacterium* in which phosphoenolpyruvate carboxykinase activity is not weakened. In other words, the microorganism of the present disclosure may be a microorganism naturally having L-glutamine production ability or a microorganism in which PEPCK or a polynucleotide encoding the same is weakened in a parent strain not having L-glutamine production ability, but is not limited thereto.

For example, a PEPCK-unmodified microorganism, in which PEPCK is not weakened and which is a target strain for comparing the increase in L-glutamine production ability, may be a wild-type *Corynebacterium glutamicum* ATCC13032 strain, a *Corynebacterium glutamicum* ATCC13032 strain having enhanced glutamine synthetase (GlnA, EC 6.3.1.2) protein activity, a microorganism in which the activity of the corresponding protein is enhanced in the microorganism by introducing mutation (D401N) into the glnA gene encoding glutamine synthetase, a *Corynebacterium glutamicum* KCCM12645P (WO2021-177731 A1) strain, a glutamine producing strain, or a *Corynebacterium glutamicum* KFCC-10680 (Korean Patent No. 10-0048440) strain, a glutamine producing strain, but is not limited thereto.

For example, the recombinant strain having increased production ability may have L-glutamine production ability increased by about 1% or more, specifically about 2% or more, about 5% or more, about 6% or more, about 7% or more, about 7.2% or more, about 8% or more, about 9% or more, or about 9.2% or more (the upper limit is not particularly limited, and may be, for example, about 200% or less, about 150% or less, about 100% or less, about 51% or less, about 40% or less, about 30% or less, about 20% or less, or about 15% or less) compared to the L-glutamine production ability of the parent strain before mutation, unmodified microorganism or PEPCK-unmodified microorganism. However, the L-glutamine production ability is not limited thereto as long as it has an increased amount of a + value compared to the production ability of the parent strain before mutation, unmodified microorganism or PEPCK-unmodified microorganism. In another example, the microorganism having increased L-glutamine production ability may have L-glutamine production ability increased by about 1.01 times or more, about 1.02 times or more, about 1.05 times or more, about 1.06 times or more, about 1.07 times or more, about 1.072 times or more, about 1.08 times or more, about 1.09 times or more, or about 1.092 times or more (the upper limit value is not particularly limited, and may be, for example, about 10 times or less, about 5 times or less, about 3 times or less, or about 2 times or less) compared to the L-glutamine production ability of the parent strain before mutation, unmodified microorganism or PEPCK-unmodified microorganism, but the L-glutamine production ability is not limited thereto.

As used herein, the term "unmodified microorganism" does not exclude strains containing mutations that may occur naturally in microorganisms, and may refer to a wild-type strain or native strain itself, or a strain before its trait is changed by genetic mutation due to natural or artificial factors. In addition, as used herein, the term "PEPCK-unmodified microorganism" may refer to a strain in which PEPCK described herein or a polynucleotide encoding the same is not weakened or a strain in which PEPCK or a polynucleotide encoding the same has not yet been weakened. The PEPCK-unmodified microorganism of the present disclosure does not exclude strains containing modifications of other proteins or genes other than the modification of PEPCK or a polynucleotide encoding the same.

As used herein, the term "unmodified microorganism" may be used interchangeably with "strain before modification", "microorganism before modification", "unmutated strain", "unmodified strain", "unmutated microorganism" or "reference microorganism".

The microorganism of the present disclosure may be a microorganism containing PEPCK of which the activity is weakened compared to the intrinsic activity or a polynucleotide encoding the same; or a microorganism (e.g., a recombinant microorganism) genetically modified to contain PEPCK of which the activity is weakened compared to the intrinsic activity or a polynucleotide encoding the same, but is not limited thereto. The "intrinsic activity" refers to the activity of a specific polypeptide originally possessed by the parent strain before transformation, wild-type or unmodified microorganism when a trait is changed by genetic mutation due to natural or artificial factors. This may be used interchangeably with "activity before modification". The fact that the activity of a polypeptide is "weakened, inactivated, deficient, decreased, down-regulated, reduced, and attenuated" compared to the intrinsic activity means that the activity of a polypeptide is lowered compared to the activity of a specific polypeptide originally possessed by the parent strain before transformation or unmodified microorganism.

As still another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris* or *Corynebacterium flavescens,* and may specifically be *Corynebacterium glutamicum,* but is not limited thereto.

As another example, the recombinant microorganism of the present disclosure may be a microorganism of which the L-glutamine production ability is enhanced by additionally enhancing the activity of some proteins in the L-glutamine biosynthesis pathway or by additionally weakening the activity of some proteins in the L-glutamine decomposition pathway.

Specifically, in the present disclosure, the microorganism of the genus *Corynebacterium* may be a microorganism (US 7262035 B2) into which the glnA enhancement (e.g. increase in copy number, promoter alteration, deregulation of activity by adenylation, weakening of glnE or weakening of PII protein activity) is additionally introduced, a microorganism (US 7262035 B2) into which glutamate dehydrogenase (GDH) enhancement (e.g., increase in copy number or modification of expression control sequence) is additionally introduced, or a microorganism (WO2021-177731 A1) into which glnA enhancing mutation is additionally introduced, but is not limited thereto.

As used herein, the term "enhancement" of the activity of a polypeptide means that the activity of the polypeptide is increased compared to the intrinsic activity. The enhancement may be used interchangeably with terms such as activation, up-regulation, overexpression, and increase. Here, activation, enhancement, up-regulation, overexpression, and increase may include both exhibiting activity that is not originally possessed, or exhibiting improved activity compared to intrinsic activity or activity before modification. The "intrinsic activity" refers to the activity of a specific polypeptide originally possessed by the parent strain before transformation or unmodified microorganism when a trait is changed by genetic mutation due to natural or artificial factors. This may be used interchangeably with "activity before modification". The fact that the activity of a polypeptide is "enhanced", "up-regulated", "overexpressed" or "increased" compared to the intrinsic activity means that the activity of a polypeptide is improved compared to the activity and/or concentration (expression level) of a specific polypeptide originally possessed by the parent strain before transformation or unmodified microorganism.

The enhancement may be achieved by introducing a foreign polypeptide or by enhancing the activity and/or concentration (expression level) of an endogenous polypeptide. Whether the activity of a polypeptide is enhanced may be determined from an increase in the activity degree or expression level of the corresponding polypeptide or an increase in the amount of a product released from the corresponding polypeptide.

For enhancement of the activity of a polypeptide, various methods well known in the art can be applied, and the method is not limited as long as the activity of the target polypeptide can be enhanced compared to that of the microorganism before modification. Specifically, the enhancement may be performed using genetic engineering and/or protein engineering, which are routine methods of molecular biology and are well known to those skilled in the art, but is not limited thereto (e.g., Sitnicka et al., Functional Analysis of Genes. Advances in Cell Biology. 2010, Vol. 2. 1-16, Sambrook et al., Molecular Cloning 2012).

Specifically, enhancement of the activity of a polypeptide of the present disclosure may be
1) an increase in intracellular copy number of a polynucleotide encoding the polypeptide;
2) replacement of the gene expression control region on the chromosome encoding the polypeptide with a highly active sequence;
3) modification of the nucleotide sequence encoding the initiation codon or 5'-UTR region of a gene transcript encoding the polypeptide;
4) modification of the amino acid sequence of the polypeptide so that the activity of the polypeptide is enhanced;
5) modification of the polynucleotide sequence encoding the polypeptide so that the activity of the polypeptide is enhanced (e.g., modification of the polynucleotide sequence of the polypeptide gene to encode a modified polypeptide so that the activity of the polypeptide is enhanced);
6) introduction of a foreign polypeptide that exhibits the activity of the polypeptide or a foreign polynucleotide encoding the same;
7) codon optimization of a polynucleotide encoding the polypeptide;
8) analysis of the tertiary structure of the polypeptide to select and modify or chemically modify an exposed site; or
9) a combination of two or more selected from 1) to 8), but is not particularly limited thereto.

More specifically,
1) the increase in intracellular copy number of a polynucleotide encoding the polypeptide may be achieved by introducing into a host cell a vector capable of replicating and functioning independently of the host, to which a polynucleotide encoding the corresponding polypeptide is operably linked. Alternatively, the increase may be achieved by introducing one copy or two or more copies of a polynucleotide encoding the corresponding polypeptide into the chromosome of the host cell. The introduction into the chromosome may be performed by introducing into the host cell a vector capable of inserting the polynucleotide into the chromosome of the host cell, but is not limited thereto. The vector is as described above.
2) The replacement of the gene expression control region (gene expression sequence) on the chromosome encoding the polypeptide with a highly active sequence may be, for example, occurrence of mutation in the sequence by deletion, insertion, non-conservative or conservative substitution, or a combination thereof, or replacement with a sequence having stronger activity so that the activity of the expression control region is further enhanced. The expression control region may include a promoter, an operator sequence, a sequence encoding a ribosome binding site, and a sequence regulating termination of transcription and translation, but is not particularly limited thereto. For example, the replacement may be to replace the original promoter with a strong promoter, but is not limited thereto.

Examples of known strong promoters include CJ1 to CJ7 promoters (US Patent No. 7662943 B2), lac promoter, trp promoter, trc promoter, tac promoter, lambda phage PR promoter, PL promoter, tet promoter, gapA promoter, SPL7 promoter, SPL13 (sm3) promoter (US Patent No. 10584338 B2), O2 promoter (US Patent No. 10273491 B2), tkt promoter, and yccA promoter, but are not limited thereto.
3) The modification of the nucleotide sequence encoding the initiation codon or 5'-UTR region of a gene transcript encoding the polypeptide may be, for example, substitution with a nucleotide sequence encoding another initiation codon having a higher polypeptide expression rate than the endogenous initiation codon, but is not limited thereto.
4) and 5) The modification of the amino acid sequence or polynucleotide sequence may be occurrence of sequence mutation in the amino acid sequence of the polypeptide or the polynucleotide sequence encoding the polypeptide by deletion, insertion, non-conservative or conservative substitution, or a combination thereof so that the activity of the polypeptide is enhanced, or replacement with an amino acid sequence or polynucleotide sequence improved to exhibit stronger activity or with an amino acid sequence or polynucleotide sequence improved to exhibit increased activity, but is not limited thereto. The replacement may be specifically performed by inserting a polynucleotide into a chromosome by homologous recombination, but is not limited thereto. The vector used at this time may further include a selection marker for determining whether the chromosome is inserted.
6) The introduction of a foreign polynucleotide that exhibits the activity of the polypeptide may be the introduction of a foreign polynucleotide encoding a polypeptide exhibiting the same/similar activity as that of the polypeptide into a host cell. The foreign polynucleotide is not limited in origin or sequence as long as it exhibits the same/similar activity as the polypeptide. The introduction may be performed by appropriately selecting and using a known transformation method by those skilled in the art. As the introduced polynucleotide is expressed in the host cell, a polypeptide may be produced and its activity may be increased.
7) The codon optimization of a polynucleotide encoding the polypeptide may be codon optimization so that transcription or translation of the endogenous polynucleotide increases in the host cell, or codon optimization of a foreign polynucleotide so that optimized transcription and translation of the foreign polynucleotide is achieved in the host cell.
8) The analysis of the tertiary structure of the polypeptide to select and modify or chemically modify an exposed site may be to determine a template protein candidate according to the degree of sequence similarity, for example, by comparing the sequence information of the polypeptide to be analyzed with a database in which sequence information of known proteins is stored, to confirm the structure based on this, to select an exposed site to be modified or chemically modified, and to modify or chemically modify the exposed site.

Such enhancement of polypeptide activity may be an increase in the activity or concentration (expression level) of the corresponding polypeptide based on the activity or concentration of the polypeptide expressed in the wild-type or microbial strain before modification, or an increase in the amount of a product produced from the corresponding polypeptide, but is not limited thereto.

Another aspect of the present disclosure provides an L-glutamine production method including culturing a microorganism of the genus *Corynebacterium* having phosphoenolpyruvate carboxykinase activity weakened and L-glutamine production ability in a medium.

The L-glutamine production method of the present disclosure may include culturing a microorganism containing PEPCK having weakened activity compared to the intrinsic activity or a polynucleotide encoding the same; or a microorganism genetically modified to contain PEPCK having weakened activity compared to the intrinsic activity or a polynucleotide encoding the same in a medium.

As used herein, the term "culture" means growing the microorganism of the present disclosure under an appropriately controlled environmental condition. The culture process of the present disclosure may be performed using suitable media under culture conditions known in the art. This culture process may be easily adjusted and used by those skilled in the art depending on the selected microorganism. Specifically, the culture may be batch, continuous and/or fed-batch culture, but is not limited thereto.

As used herein, the term "medium" refers to a material in which nutrients required for culturing the microorganism of the present disclosure are mixed as main components, and supplies nutrients and growth factors, including water essential for survival and growth. Specifically, as the medium and other culture conditions used for culturing the microorganism of the present disclosure, any medium may be used without particular limitation as long as it is a medium used for culturing common microorganisms. The microorganism of the present disclosure may be cultured in a conventional medium containing appropriate carbon sources, nitrogen sources, phosphorus sources, inorganic compounds, amino acids, and/or vitamins under aerobic conditions while the temperature, pH, and the like are controlled.

Specifically, culture media for microorganisms of the genus *Corynebacterium* may be found in the literature ["Manual of Methods for General Bacteriology" by the American Society for Bacteriology (Washington D.C., USA, 1981)].

In the present disclosure, examples of the carbon sources include carbohydrates such as glucose, saccharose, lactose, fructose, sucrose, and maltose; sugar alcohols such as mannitol and sorbitol; organic acids such as pyruvic acid, lactic acid, and citric acid; and amino acids such as glutamic acid, methionine, and lysine. Natural organic nutritional sources such as starch hydrolysate, molasses, blackstrap molasses, rice bran, cassava, sugarcane bagasse and corn steep liquor may be used. Specifically, carbohydrates such as glucose and sterilized and pretreated molasses (i.e., molasses converted to reducing sugar) may be used, and appropriate amounts of other carbon sources may be variously used without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and defatted soybean cake or decomposition products thereof may be used. These nitrogen sources may be used singly or in combination of two or more thereof, but are not limited thereto.

Examples of the phosphorus sources may include monopotassium phosphate and dipotassium phosphate or monosodium phosphate and disodium phosphate. As the inorganic compounds, sodium chloride, calcium chloride, iron chloride, magnesium sulfate, iron sulfate, manganese sulfate, calcium carbonate, and the like may be used, and amino acids, vitamins, appropriate precursors and/or the like may be used in addition to these. These components or precursors may be added to the medium either batchwise or continuously, but the manner to add the components or precursors is not limited thereto.

During the culture of the microorganism of the present disclosure, compounds such as ammonium hydroxide, potassium hydroxide, ammonia, phosphoric acid, and sulfuric acid may be added to the medium in an appropriate manner to adjust the pH of the medium. During culture, the formation of bubbles may be suppressed by using antifoaming agents such as fatty acid polyglycol esters. Oxygen or oxygen-containing gas may be injected into the medium in order to maintain the aerobic state of the medium, or gas may not be injected or nitrogen, hydrogen, or carbon dioxide gas may be injected in order to maintain anaerobic and microaerobic conditions, but the atmosphere controlling method is not limited thereto.

In the culture of the present disclosure, the culture temperature may be maintained at 20°C to 45°C, specifically 25°C to 40°C, and the culture time may be about 10 to 160 hours, but the culture conditions are not limited thereto.

L-glutamine produced by the culture of the present disclosure may be secreted into the medium or remain in the cells.

The L-glutamine production method of the present disclosure may further include preparing the microorganism of the present disclosure, preparing a medium for culturing the microorganism, or a combination thereof (in any order), for example, prior to the culturing step.

The L-glutamine production method of the present disclosure may further include recovering L-glutamine from the cultured medium (medium subjected to culture) or the cultured microorganism. The recovering step may be further included after the culturing step.

The recovery may be to collect the desired L-glutamine using a suitable method known in the art according to the method for culturing a microorganism of the present disclosure, for example, batch, continuous or fed-batch culture method. For example, centrifugation, filtration, treatment (salting out) with a precipitating agent for crystallized proteins, extraction, sonication, ultrafiltration, dialysis, various kinds of chromatography such as molecular sieve chromatography (gel filtration), adsorption chromatography, ion exchange chromatography, and affinity chromatography, HPLC, or a combination thereof may be used. The desired L-glutamine may be recovered from the medium or microorganism by suitable methods known in the art.

The L-glutamine production method of the present disclosure may further include a purification step. The purification may be performed by suitable methods known in the art. In an example, when the L-glutamine production method of the present disclosure includes both a recovery step and a purification step, the recovery step and the purification step may be performed continuously or discontinuously regardless of order, or may be performed simultaneously or by being integrated into one step, but the manner to perform the recovery step and the purification step is not limited thereto.

In the method of the present disclosure, PEPCK, polynucleotide, vector and microorganism, and the like are as described in the other aspects.

Still another aspect of the present disclosure provides a composition for L-glutamine production containing a microorganism in which phosphoenolpyruvate carboxykinase is weakened; a medium in which the microorganism has been cultured; or a combination thereof.

The composition of the present disclosure may further contain any suitable excipient commonly used in compositions for L-glutamine production, and such an excipient may be, for example, preservatives, wetting agents, dispersing agents, suspending agents, buffers, stabilizers, or tonicity agents, but are not limited thereto.

Still another aspect of the present disclosure provides a method for producing a microorganism for L-glutamine production, including weakening PEPCK.

Still another aspect of the present disclosure provides use of a microorganism of the genus *Corynebacterium* for L-glutamine production, in which phosphoenolpyruvate carboxykinase is weakened.

The PEPCK, weakening, microorganism of the genus *Corynebacterium,* and the like are as described in the other aspects.

### [Examples]

Hereinafter, the present disclosure will be described in more detail with reference to Examples. However, the following Examples are merely preferred embodiments for illustrating the present disclosure and, therefore, are not intended to limit the scope of the present disclosure thereto. Meanwhile, technical matters not described in the present specification can be sufficiently understood and easily implemented by those skilled in the art of the present disclosure or similar technical fields.

### Example 1: Construction of vector for weakening pck gene

In order to weaken the pck gene encoding phosphoenolpyruvate carboxykinase, a vector for deleting the gene was constructed.

Specifically, in order to construct a strain in which the pck gene portion is deleted, PCR was performed using the chromosome of *Corynebacterium glutamicum* ATCC13032 as a template and the primers of SEQ ID NO: 3 and SEQ ID NO: 4, SEQ ID NO: 1 and SEQ ID NO: 2, respectively. The primer sequences used here are shown in Table 1 below.

**[Table 1]**

| SEQ ID NO: | Name | Sequence |
|---|---|---|
| 3 | primer 1 | ggggatcctctagagtcgacCTGCGACGACTGGAAAACCATGG |
| 4 | primer 2 | GCAGTTCTTAAGCGTGAACTactagtTAAAACTTTAGGTGAGACAAC |
| 5 | primer 3 | GTTGTCTCACCTAAAGTTTTAactagtAGTTCACGCTTAAGAACTGC |
| 6 | primer 4 | gcttgcatgcctgcaggtcgacGGCTGGACCCTAGAATTCGG |

As a polymerase for the PCR reaction, PfuUltra^{™} high-reliability DNA polymerase (Stratagene) was used. As the PCR condition, denaturation at 95°C for 30 seconds; annealing at 55°C for 30 seconds; and polymerization reaction at 72°C for 1 minute were repeated 28 times. As a result, a 929 bp DNA fragment at the 5' upstream end centered on the pck gene initiation codon and a 1000 bp DNA fragment at the 3' downstream end centered on the pck gene termination codon were obtained, respectively. The two amplified DNA fragments were purified using a PCR purification kit (QUIAGEN) and used as insert DNA fragments for plasmid construction. Meanwhile, cloning was performed by setting the molar concentration (M) ratio of the pDZ vector (Korean Patent No. 2009-0094433) subjected to treatment with the restriction enzyme Sall and heat treatment at 65°C for 20 minutes to the insert DNA fragment amplified through PCR to 1 : 2 and using the Infusion Cloning Kit (TaKaRa) according to the provided manual to construct vector pDZ-ΔPEPCK for pck gene deletion.

### Example 2: Construction of wild-type based strain of genus Corynebacterium having L-glutamine production ability

An L-glutamine producing strain was developed from wild-type *Corynebacterium glutamicum* ATCC13032. Specifically, in order to increase the activity of glutamine synthetase, a biosynthesis pathway terminal enzyme, a strain (Accession No. KCCM12645P) into which glnA (D401N) mutant (SEQ ID NO: 11), a gene encoding glutamine synthetase, was introduced was constructed.

Specifically, in order to construct a strain into which glnA (D401N) mutation was introduced, PCR was performed using the chromosome of *Corynebacterium glutamicum* ATCC13032 as a template and the primers of SEQ ID NO: 7 and SEQ ID NO: 8 or SEQ ID NO: 9 and SEQ ID NO: 10, respectively. The primer sequences used here are shown in Table 2 below.

**[Table 2]**

| SEQ ID NO: | Name | Sequence |
|---|---|---|
| 7 | primer 5 | ggggatcctctagagtcgacgtggcgtttgaaaccccggaag |
| 8 | primer 6 | catcgagccacacgctccagtgaacaaggacctctacgaac |
| 9 | primer 7 | gttcgtagaggtccttgttcactggagcgtgtggctcgatg |
| 10 | primer 8 | gcttgcatgcctgcaggtcgacttagcagtcgaagtacaattcg |

As a polymerase for the PCR reaction, PfuUltra^{™} high-reliability DNA polymerase was used. As the PCR condition, denaturation at 95 °C for 30 seconds; annealing at 55°C for 30 seconds; and polymerization reaction at 72°C for 1 minute were repeated 28 times. As a result, a DNA fragment at the 5' upstream end and a DNA fragment at the 3' downstream end centered on the glnA gene D401N mutation were obtained, respectively. The two amplified DNA fragments were purified using a PCR purification kit and used as insert DNA fragments for plasmid construction. Meanwhile, cloning was performed by setting the molar concentration (M) ratio of the pDZ vector (Korean Patent No. 2009-0094433) subjected to treatment with the restriction enzyme spel and heat treatment at 65°C for 20 minutes to the insert DNA fragment amplified through PCR to 1 : 2 and using the Infusion Cloning Kit according to the provided manual to construct pDZ-glnA(D401N) for inserting glnA(D401N) gene mutant.

The constructed vector was transformed into *Corynebacterium glutamicum* ATCC13032 by electroporation and electropulsing (Van der Rest et al., Appl. Microbial. Biotechnol. 52:541-545, 1999) to obtain a strain containing glnA (D401N) mutation on the chromosome by homologous chromosomal recombination. The strain was named *Corynebacterium glutamicum* CA11-4021, and then deposited with the Korean Culture Center of Microorganisms (KCCM), an international depository institution under the Budapest Treaty, on December 19, 2019, and was given Accession Number KCCM12645P.

### Example 3: Construction and evaluation of L-glutamine producing strain having pck gene weakened

The pck gene in *Corynebacterium glutamicum* CA11-4021, an L-glutamine producing strain constructed in Example 2, was weakened. More specifically, the vector constructed in Example 1 was transformed into *Corynebacterium glutamicum* CA11-4021 by electroporation, and a second crossover was performed to obtain an L-glutamine producing strain in which the pck gene is deleted on the chromosome, and was named *Corynebacterium glutamicum* CA11-4023.

The glutamine production ability was measured by culturing *Corynebacterium glutamicum* CA11-4023 in the following manner.

First, each strain was inoculated into a 250 ml corner-baffled flask containing 25 ml of seed medium, and shaking-cultured at 200 rpm at 30°C for 20 hours. Next, 1 ml of the seed culture solution was inoculated into a 250 ml corner-baffled flask containing 24 ml of production medium, and shaking-cultured at 200 rpm at 32°C for 48 hours. The compositions of the seed medium and production medium are as follows, respectively. After completion of the culture, the concentration of L-glutamine was measured using HPLC (Waters 2478). The measurement results of glutamine production ability and sugar consumption rate are as shown in Table 3 below.

### <Seed medium (pH 7.0)>

Glucose 20 g, peptone 10 g, yeast extract 5 g, urea 1.5 g, KH₂PO₄ 4 g, K₂HPO₄ 8 g, MgSO₄ 7H₂O 0.5 g, biotin 100 µg, thiamine HCl 1000 µg, calcium-pantothenic acid 2000 µg, and nicotinamide 2000 µg (based on 1 liter of distilled water)

### <Glutamine production medium (pH 8.0)>

Raw sugar 60 g, (NH₄)₂SO₄ 45 g, soybean protein 0.48 g, CaCOs 50 g, MgSO₄ 7H₂O 0.4 g, KH₂PO₄ 1 g, thiamine hydrochloride 0.2 mg, biotin 0.3 mg, nicotinamide 60 mg, FeSO₄ 7H₂O 10 mg, and MnSO₄ H₂O 10 mg (based on 1 liter of distilled water)

**[Table 3]**

| Strain | Genetic trait | L-glutamine (g/l) |
|---|---|---|
| ATCC13032 | | 0.89 |
| CA11-4021 | ATCC13032::glnA(D401N) | 1.25 |
| CA11-4023 | CA11-4021△PEPCK | 1.34 |

As a result, it was confirmed that the glutamine production ability of CA11-4023 in which the pck gene is weakened was improved by 7.2% compared to that of CA11-4021, the parent strain.

The CA11-4023 strain was deposited with the Korean Culture Center of Microorganisms (KCCM), an international depository institution under the Budapest Treaty, on December 22, 2020, and was given Accession Number KCCM 12916P.

### Example 4: Construction and evaluation of high-concentration L-glutamine producing strain having pck gene weakened

The pck gene in the *Corynebacterium glutamicum* KFCC-10680 (Korean Patent No. 10-0048440) strain, a known glutamine producing strain, was weakened in the same manner as in Example 3. More specifically, the pDZ-ΔPEPCK vector constructed in Example 1 was transformed into *Corynebacterium glutamicum* KFCC-10680 by electroporation, and a second crossover was performed to obtain a strain in which the pck gene is deleted on the chromosome was obtained, was named *Corynebacterium glutamicum* KFCC-10680Δpck, and was cultured in the following manner to measure the glutamine production ability.

First, each strain was inoculated into a 250 ml corner-baffled flask containing 25 ml of seed medium, and shaking-cultured at 200 rpm at 30°C for 20 hours. Next, 1 ml of the seed culture solution was inoculated into a 250 ml corner-baffled flask containing 24 ml of production medium, and shaking-cultured at 200 rpm at 32°C for 48 hours. The compositions of the seed medium and production medium are as follows, respectively. After completion of the culture, the concentration of L-glutamine was measured using HPLC (Waters 2478). The measurement results of glutamine production ability and sugar consumption rate are as shown in Table 4 below.

### <Seed medium (pH 7.0)>

Glucose 20 g, peptone 10 g, yeast extract 5 g, urea 1.5 g, KH₂PO₄ 4 g, K₂HPO₄ 8 g, MgSO₄ 7H₂O 0.5 g, biotin 100 µg, thiamine HCl 1000 µg, calcium-pantothenic acid 2000 µg, and nicotinamide 2000 µg (based on 1 liter of distilled water)

### <Glutamine production medium (pH 8.0)>

Raw sugar 60 g, (NH₄)₂SO₄ 45 g, soybean protein 0.48 g, CaCOs 50 g, MgSO₄ 7H₂O 0.4 g, KH₂PO₄ 1 g, thiamine hydrochloride 0.2 mg, biotin 0.3 mg, nicotinamide 60 mg, FeSO₄ 7H₂O 10 mg, and MnSO₄ H₂O 10 mg (based on 1 liter of distilled water)

**[Table 4]**

| Strain | L-glutamine (g/L) |
|---|---|
| KFCC-10680 | 13.8 |
| KFCC-10680△pck | 15.4 |

As a result, it was confirmed that the glutamine production ability of KFCC-10680Δpck in which the pck gene is deleted was improved by 9.2% compared to that of KFCC-10680, the parent strain.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes or modified forms derived from the meaning and scope of the claims and their equivalent concepts should be construed as being included in the scope of the present disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA11-4021 | Accession number given by the
INTERNATIONAL DEPOSITARY AUTHORITY:
KCCM12645P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on December. 19. 2019 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2019.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
Corynebacterium glutamicum CA11-4023 | Accession number given by the
INTERNATIONAL DEPOSITARY AUTHORITY:
KCCM12916P

II. SCIENTIFIC DESCRIPTION AND/OR PROPOSED TAXONOMIC DESIGNATION

The microorganism identified under I above was accompanied by:
☐ a scientific description
☐ a proposed taxonomic designation
(Mark with a cross where applicable)

III. RECEIPT AND ACCEPTANCE

This International Depositary Authority accepts the microorganism identified under I above,
which was received by it on December. 22. 2020 (date of the original deposit).¹

IV. RECEIPT OF REQUEST FOR CONVERSION

The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY

Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 22. 2020.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yurim Bldg., 45, Hongjenae 2ga-gil, Seodaemun-gu, Seoul, 03641, Korea Tel: 82-2-391-0950, 396-0950 Fax: 82-2-392-2859

## Claims

1. A microorganism of the genus *Corynebacterium* having phosphoenolpyruvate carboxykinase activity weakened and L-glutamine production ability.

2. The microorganism according to claim 1, wherein the microorganism has increased L-glutamine production ability compared to a parent strain or wild-type strain of the genus *Corynebacterium* in which phosphoenolpyruvate carboxykinase activity is not weakened.

3. The microorganism according to claim 1, wherein the phosphoenolpyruvate carboxykinase is an intrinsic protein.

4. The microorganism according to claim 1, wherein the microorganism is *Corynebacterium glutamicum.*

5. The microorganism according to claim 1, wherein the phosphoenolpyruvate carboxykinase consists of an amino acid sequence of SEQ ID NO: 1 or an amino acid sequence having 90% or more identity to the amino acid sequence of SEQ ID NO: 1.

6. An L-glutamine production method comprising culturing the microorganism according to any one of claims 1 to 5 in a medium.

7. The L-glutamine production method according to claim 6, further comprising recovering L-glutamine from the medium or microorganism after the culturing step.

8. Use of a microorganism of the genus *Corynebacterium* for L-glutamine production, wherein the microorganism has weakened phosphoenolpyruvate carboxykinase.
